# EUROPEAN PATENT APPLICATION

(11) **EP 1 625 830 A1**
(43) Date of publication of application: **15.02.2006**
(21) Application number: 04734079.9
(22) Date of filing: 20.05.2004
(51) Int. Cl.: A61B 19/00

(54) **MEDICAL MENIPULATOR**

(30) Priority: 22.05.2003 JP 2003145056
(71) Applicant: WASEDA UNIVERSITY, Tokyo 169-8050 (JP); Fujie, Masakatsu, Tokyo 169-8555 (JP)
(72) Inventor: FUJIE, Masakatsu, Waseda Uni.,School Sci. & Engin., Tokyo 169-8555 (JP); OKAYASU, H. c/o School of Science and Engineering, Shinjuku-ku, Tokyo 1698555 (JP)
(74) Representative: Grünecker, Kinkeldey, Stockmair & Schwanhäusser Anwaltssozietät
(86) International application number: PCT/JP2004/006824
(87) International publication number: WO 2004/103197

(57) **Abstract**

A medical manipulator (10), comprising a plurality of manipulator formed bodies (14) to (18) longitudinally connected to each other so as be displaced relative to each other. The manipulator formed bodies (14) to (18) further comprise bases (20) and balloon bodies (21) installed on the bases (20) and providing pressing forces to structures in a living body. The balloon bodies (21) further comprise spaces (48) capable of storing normal saline solution supplied from a device body (12), and installed so that the pressing forces can be controlled by controlling internal pressures in the spaces (48). The manipulator (10) is so formed as to be autonomously moved forward in the living body by the control of the pressing force while the manipulator formed bodies (14) to (18) are displaced relative to each other by a reaction from the structures in the living body.

## Description

### TECHNICAL FIELD

This invention relates to a medical manipulator, and more specifically, to a medical manipulator that can autonomously advance in a living body under a reaction force from tissues in the living body and without the need for a propulsive force exerted by a robot or the like.

### BACKGROUND ART

A medical manipulator system is now prevailing which is used to achieve minimally invasive surgery that do not involve a large incision (see Patent Document 1). This medical manipulator system comprises a manipulator provided with a surgical instrument such as an endoscope or a treatment instrument at its front end and a robot arm that grips the manipulator to move it in a predetermined direction. With this system, an operator remotely operates the robot arm to insert the manipulator into the body. The operator then uses the instrument provided at the front end to for example, treat the affected part. The instrument provided to be able to bend by using a servo motor as a driving source to pull a plurality of wires connected to the front end. Further, with this system, when the manipulator runs out of control, the operator activates a switch placed at his or her feet to shut down the system. The operator can thus forcibly stop the operation of the manipulator.
[Patent Document 1] Japanese Patent Publication No. 9-66056

However, since the medical manipulator system uses the robot arm to move the manipulator, if the robot runs out of control, it is inconveniently impossible to ensure that inadvertent movement of the manipulator can be prevented. That is, to stop the manipulator emergently when it runs out of control, the operator must activate the switch. To perform this operation, the operator must recognize that the manipulator is running out of control. During an operation, the manipulator is likely to be present in the body. Accordingly, it is difficult for the operator to see the manipulator running out of control. This delays the finding of the running out of control to prevent the manipulator from being stopped immediately after the occurrence of the running out of control. Further, if the operator quickly recognizes the occurrence of the running out of control and immediately activates the switch, the driving actuator for the robot arm is stopped. However, owing to its inertia or the like, the manipulator is stopped slightly later than the driving actuator. This is a serious problem for the manipulator, which is inserted into the body, which provides the manipulator with a narrow operational range. The delay in stop may cause body tissues to be damaged.

Further, the above system uses a complicated mechanism employing a metal wire, a servo motor, or the like, to provide to be able to bend the manipulator. This precludes the safety of the living body from being ensured when the mechanism is broken. Further, it is necessary to complicate the whole system and to increase the size of the system. That is, if the manipulator inserted into the body was broken, electrical leak might occur through the wire. Furthermore, since the driving source is the servo motor, if a magnetic resonance imaging system (MRI) is used with the manipulator, the servo motor must be separated sufficiently from the MRI. This is to prevent the MRI from affecting by the driving of the servo motor. That is, a main body including the servo motor must be separated sufficiently from the manipulator, located in proximity to the MRI. This requires a large space in which the whole system is installed. Moreover, the above system requires a wire winding mechanism. Accordingly, if the manipulator has a multijoint structure, the winding mechanism must further be complicated in order to wind each wire independently. This increases the number of components required in the whole system. Therefore, the system structure must be complicated.

### DISCLOSURE OF THE INVENTION

The present invention is originated by taking notice of the above inconveniences. It is an object of the present invention to provide a medical manipulator that can be autonomously advanced in a living body without the need to exert a propulsive force caused by a robot or the like, and that manipulator is thus prevented from running out of control.

It is another object of the present invention to provide a medical manipulator which can keep a living body safe even when the manipulator is broken, and which enables to be simplified and the configuration of the whole system to facilitate a reduction in the size of the system.

To attain these objects, the present invention provides a medical manipulator comprising a number of manipulator formed bodies connected together in a longitudinal direction so as to be relatively displaceable, the manipulator advancing in a loving body,
wherein at least some of the manipulator formed bodies comprise a base and a pressure section provided on the base to exert a pressing force on tissues in the living body,
the pressure section comprises a space in which a predetermined fluid can be accommodated, the pressing force can be adjusted by regulating an internal pressure of the space, and adjustment of the pressing force allows the manipulator formed bodies to advance autonomously in the living body while being relatively displaced under a reaction force from the tissues.
With this configuration, when the manipulator advances through a gap in the living body, the manipulator formed bodies are relatively displaced under the reaction force from the tissues present around the gap. Consequently, the interaction with the tissues allows the manipulator to advance autonomously like a looper while following the shape of the gap. This eliminates the need for a robot that forces the manipulator to move in spite of the presence of the tissues. It is thus possible to preclude inadvertent movement of the manipulator resulting from the running out of control of the robot. Further, since the manipulator formed bodies are relatively displaced under the reaction force from the tissues, the pressure section is prevented from exerting an unnatural pressing force on the tissues. Therefore, the manipulator can be used even for the brain, which is composed of soft tissues. Moreover, while the manipulator is advancing, the manipulator formed bodies are in a free state (passively controlled state) and can be relatively displaced. This allows the manipulator to be easily pulled out of the living body even if the system fails. Further, it is unnecessary to provide any wires, motors, sensors, or other mechanisms that relatively displace the manipulator formed bodies. This makes it possible to avoid electrical leak through wires when the manipulator is broken, thus improving safety. Further, the entire configuration of the system including the manipulator can be simplified to facilitate a reduction in the size of the system. Moreover, the supply of a fluid allows the manipulator formed bodies to be relatively displaced. Consequently, if a magnetic resonance imaging system (MRI) is used with the manipulator, the manipulator can suppress the adverse effect on MRI. Further, owing to the correlation between the pressing force on the tissues and the internal pressure of the space, the pressing force on the tissues can be detected by measuring the internal pressure of the space. Therefore, the manipulator can be allowed to function as a pressure sensor.

According to the present invention, the pressure section is preferably composed of a balloon body that expands and deflates in response to an increase and decrease in the amount of fluid accommodated.
This makes it possible to enlarge the gap in the living body when the manipulator advances through the living body. The manipulator can be advanced while pushing through the gap. Further, the pressure section can be made flexible enough to prevent an excessive pressing force from acting on the tissues constituting the periphery of the gap.

Furthermore, preferably, the pressure section is provided in each of the manipulator formed bodies and the pressure force of each pressure section is independently adjustable.
This enables the position of the manipulator to be complicatedly displaced. Consequently, the manipulator can be easily advanced even if the gap in the living body through which the manipulator is inserted has a complicated curved shape or the like.

Moreover, preferably, the pressure section is provided on both a upper and lower surfaces of base so as to press the tissues upward and downward with respect to the base.
This arrangement enables the manipulator to advance autonomously while exerting reduced pressing forces with the upward and downward pressing forces balanced.

Further, preferably, a channel is formed in the base so that the channel is in communication with a space in the pressure section.
This arrangement eliminates the need for lines such as external tubes which are used to supply a fluid to the space in the pressure section. This simplifies the configuration of the manipulator.

In this case, connecting means may be provided between the bases to connect the bases so that the bases can be relatively displaced, and a connecting passage may be formed in the connecting means to allow said channels formed in the bases to be in communication with each other.
This also simplifies the configuration of the manipulator to enable the manipulator to advance smoothly.

In this case, the connection passage is configured to keep the channels in communication with each other even when the bases are relatively displaced.
With this arrangement, even if the area of engaging parts of the connecting means decreases when the manipulator formed bodies are relatively displaced, channels through which a fluid is supplied to the space, can be provided. This makes it possible to maintain the continuous advancement of the manipulator.

In this case, preferably, the fluid is normal saline solution, and the connecting passage allows leakage of part of the normal saline solution passing through the connecting passage so as to supply the normal saline solution to the interior of the living body.
With this arrangement, the normal saline solution can be supplied to the living body during an operation or the like without the need to insert for example, separate tubes for droplet into the living body when the manipulator advances through the living body. This minimizes the number of manipulators inserted into the gap in the living body. Therefore, the present invention is useful even if the gap is narrow.

Further, restricting means are provided between the manipulator formed bodies so as to restrict the relative displacement of the manipulator formed bodies. Thus, in the simple configuration of the manipulator, it can be locked in any positions.

In the description, the term "front" means a leading end of the manipulator in its advancing direction and the term "rear" means the opposite side unless otherwise specified. The term "width direction" means a direction orthogonal to the advancing direction of the manipulator as seen from above. If not otherwise specified, the terms "upper" and "lower" mean one and the other ends of two directions mutually orthogonal to the advancing direction of the manipulator which direction does not correspond to the width direction.

### BEST MODE FOR CARRYING OUT THE INVENTION

Embodiments of the present invention will be described below with reference to the drawings.

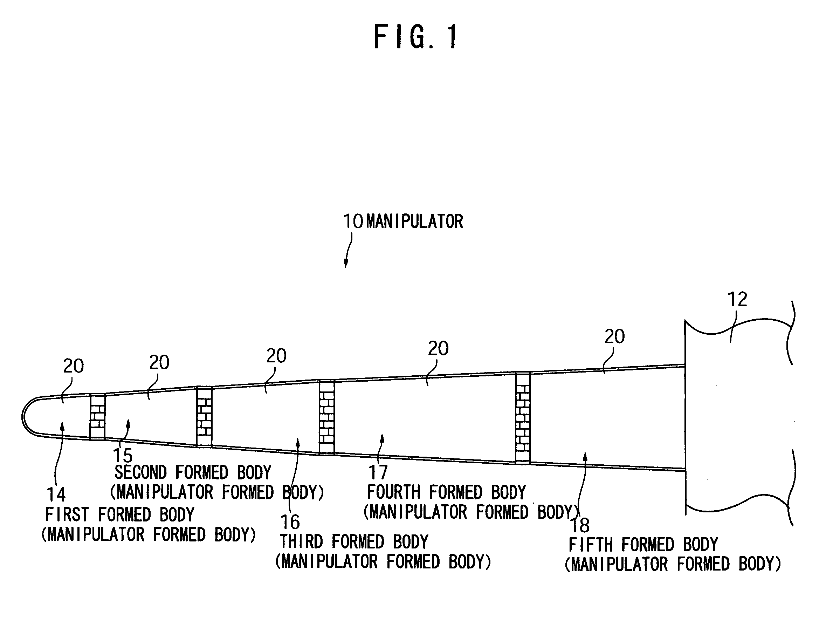

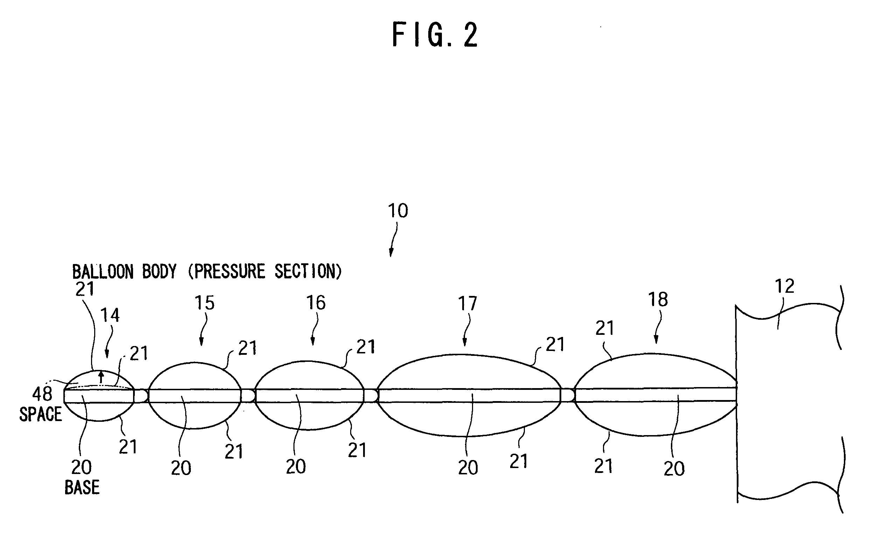

Figure 1 shows a schematic plan view of a medical manipulator in accordance with the present embodiment. Figure 2 is a schematic side view of the manipulator. In these figures, a manipulator 10 is a leading manipulator that enlarges a gap in a living body. The manipulator 10 allows an instrument manipulator (not shown) to be easily inserted into the living body; the instrument manipulator is provided with a surgical instrument, e.g., a scalpel at its front end. The manipulator 10 has its rear end held by an apparatus main body 12. The manipulator 10 consists of five manipulator formed bodies 14 to 18 connected together in a longitudinal direction so as to be relatively displaceable. In the description below, the manipulator formed bodies 14 to 18 are called a first formed body 14, a second formed body 15, a third formed body 16, a fourth formed body 17, and a fifth formed body 18.

The first formed body 14 comprises a base 20, and balloon bodies 21 (see Figure 2) provided over and under the base 20 and serving as pressure sections that exert pressure forces to tissues in the living body when the manipulator is inserted into the living body.

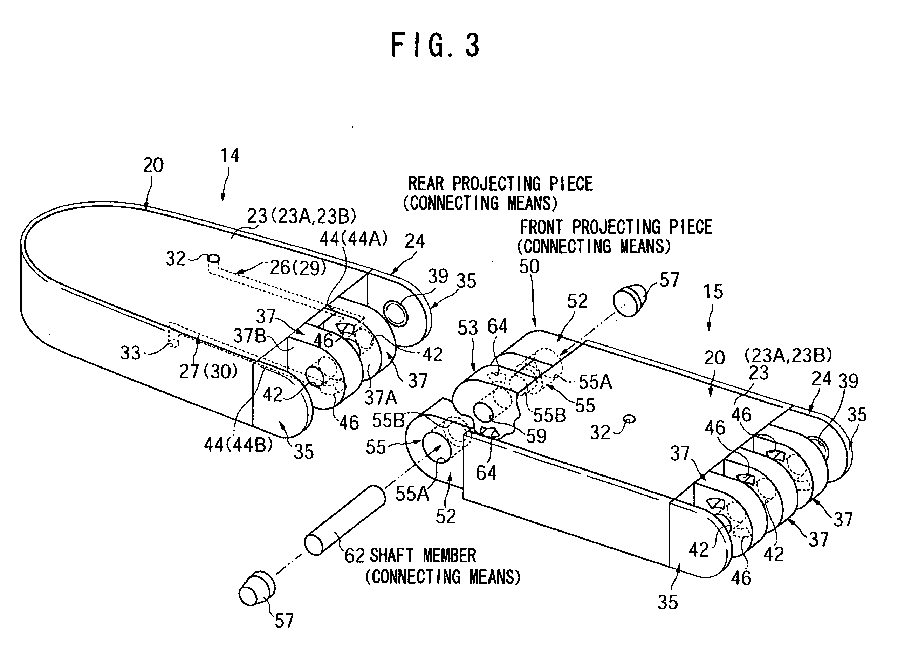

As shown in Figures 3 to 5, the base 20 comprises a main body 23 shaped like an elongate piece having a curved front edge, and a rear projecting piece 24 that projects rearward from a rear end of the main body 23.

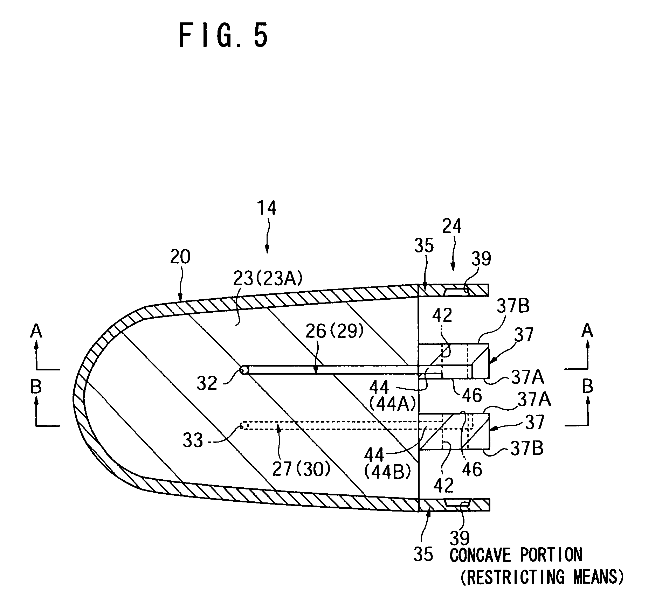

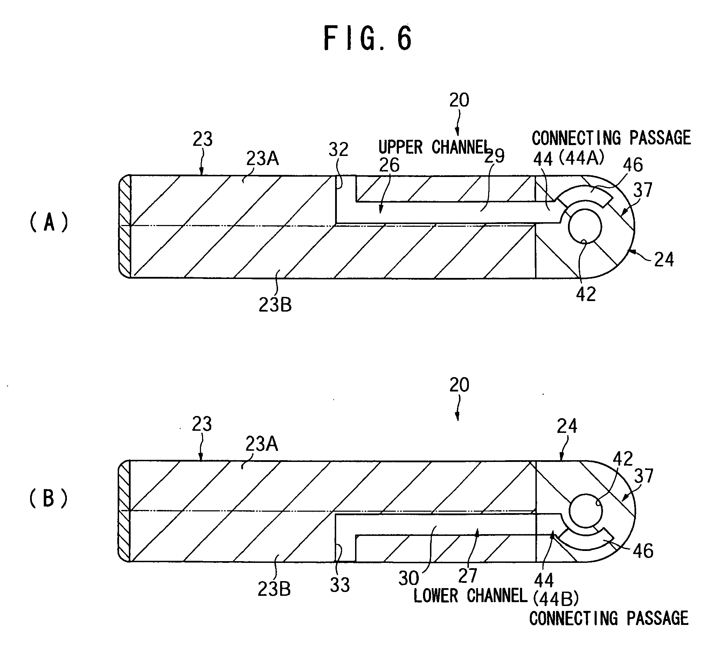

As shown in Figure 6, the main body 23 consists of an upper area 23A constituting nearly the upper half and a lower area 23B constituting nearly the lower half. An upper channel 26 and a lower channel 27 are formed inside the upper area 23A and lower area 23B, respectively, so that normal saline solution supplied by the apparatus main body 12 (see Figure 1 and the like) passes through the upper channel 26 and lower channel 27. As shown in Figures 5 and 6, the upper channel 26 consists of one upper first supply channel 29 extended in the longitudinal direction. The upper first supply channel 29 is in communication with an upper supply hole 32 that is open on an upper surface of the main body 23. On the other hand, the lower channel 27 consists of one lower first supply channel 30 extended in the longitudinal direction along a position where it does not interfere with the upper first supply channel 29. The lower first supply channel 30 is in communication with a lower supply hole 33 that is open on a lower surface of the main body 23.

As shown in Figures 3 to 6, the rear projecting piece 24 consists of a pair of rear side pieces 35, 35 located at the both ends in the width direction and two rear intermediate pieces 37, 37 located between the rear side pieces 35, 35. The rear side pieces 35 and rear intermediate pieces 37 are not particularly limited but are formed so as to have an external shape linearly extending rearward from their base end and having a semicircular rear end.

Each rear side piece 35 is provided with an inwardly open concave portion 39 located virtually in the center of the rear side piece. The concave portion 39 has a virtually circular external shape in a front view. The concave portion 39 is shaped like a tapered hole having an internal dimension decreasing gradually from its open side toward the interior as shown in Figure 5.

Each of the rear intermediate piece 37 comprises an inner side surface 37A and an outer side surface 37B located at the both ends in the width direction, a shaft insertion hole 42 penetrating the rear intermediate piece 37 virtually in its center in the width direction, and a connecting passage 44 that is in communication with the upper channel 26 and lower channel 27 at positions where the connecting passage 44 does not interfere with the shaft insertion hole 42. The term "inner side surface 37A" means a side surface facing the another rear intermediate piece 37. The term "outer side surface 37B" means a side surface lying opposite the inner side surface 37A.

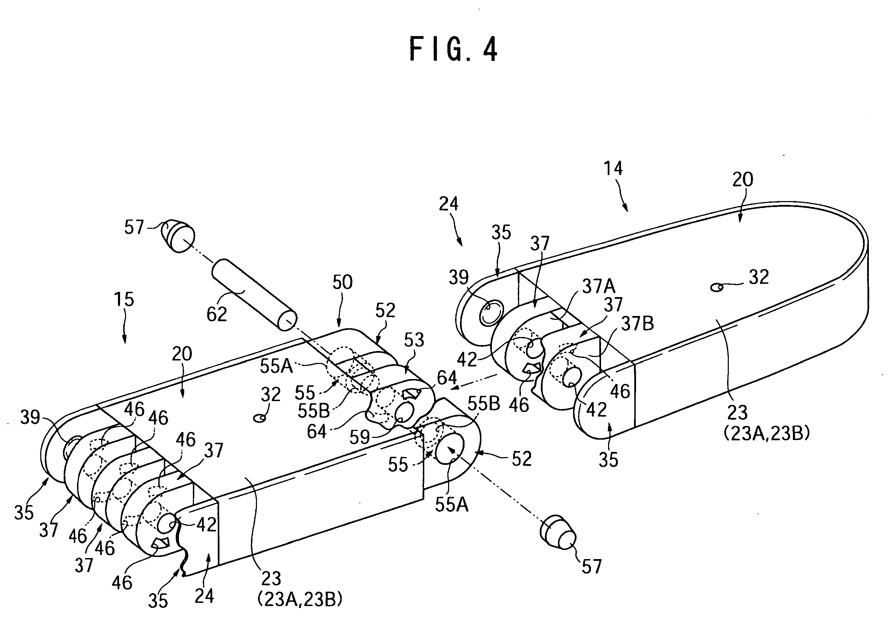

The connecting passage 44 consists of an upper connecting passage 44A formed in an upper area of the rear intermediate piece 37 located in the upper side of Figure 5, and a lower connecting passage 44B formed in a lower area of the rear intermediate piece 37 located in the lower side of Figure 5. The upper connecting passage 44A is in communication with the upper first supply channel 29, formed in the upper area 23A of the main body 23. The upper connecting passage 44A extends rearward from the upper first supply passage 29 and bends to the width direction in the middle of the passage. The upper connecting passage 44A is thus open on the inner side surface 37A. On the other hand, the lower connecting passage 44B is in communication with the lower first supply channel 30, formed in the lower area 23B of the main body 23. The lower connecting passage 44B extends rearward from the lower first supply passage 30 and bends to the width direction in the middle of the passage. The lower connecting passage 44B is thus open on the inner side surface 37A. As shown in Figures 3 and 4, open portions 46 of the upper connecting passage 44A and lower connecting passage 44B constitute slots having an external shape similar to fans and are formed to have virtually the same shape and size. Further, as shown in Figure 6, the open portions 46 are formed to be symmetrical in the lateral and to be symmetrical in vertical directions between the figure6 (A) and 6(B).

As shown in Figure 2, the balloon bodies 21 are fixed along each periphery of upper and lower surfaces of the base 20. The interiors of the balloon bodies 21 constitute spaces 48 in which the normal saline solution flowing out through the upper supply hole 32 and the lower supply hole 33 (see Figure 3) is accommodated. The balloon bodies 21 are formed of an elastic material such as latex which has a high biocompatibility. The balloon bodies 21 expand and deflate in response to an increase and decrease in the amount of the normal saline solution accommodated. In its initial state in which no normal saline solution is accommodated in the space 48, each of the balloon bodies 21 is placed like a sheet along a surface of the base 20 as shown by a chain double-dashed line in the figure. On the other hand, as the normal saline solution is supplied to the space 48, the balloon body 21 inflates away from the base 20 as shown by a solid line in Figure 2.

Now, the second to fifth formed bodies 15 to 18 will be described. Almost all the formed bodies 15 to 18 have components that are substantially the same as or equivalent to those of the first formed body 14. The same or equivalent components are denoted by the same reference signs and their description will be omitted or be brief.

As shown in Figures 3, 4, and 7, the second formed body 15 further comprises a front projecting piece 50 projecting frontward from a front end of the main body 23. The front projecting piece 50 is shaped so that it can engage with the rear projecting piece 24 of the first formed body 14. That is, the projecting piece 50 consists of a pair of front side pieces 52, 52 received in the gap between the rear side piece 35 and the rear intermediate piece 37, and a front intermediate piece 53 received in the gap between the opposite rear intermediate pieces 37, 37. The front side pieces 52 and the front intermediate pieces 53 are not particularly limited but are formed so as to have an external shape linearly extending frontward from their base end and having a semicircular front end.

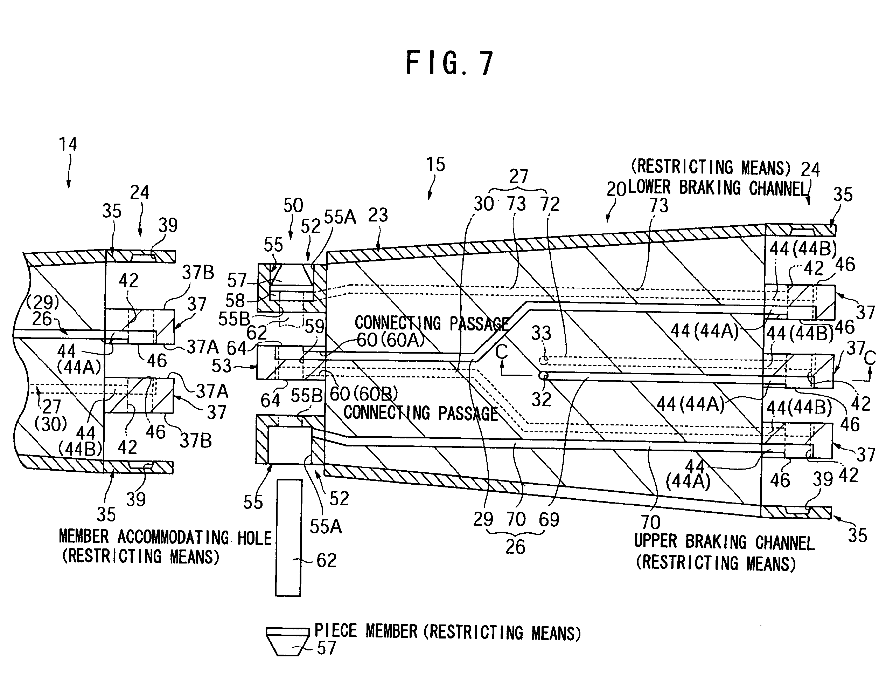

A member accommodating hole 55 is formed in the front side piece 52 so as to penetrate the front side piece 52 in the width direction virtually in its center. The member accommodating hole 55 is shaped like a stepped hole consisting of a larger diameter portion 55A located outside in the width direction and a smaller diameter portion 55B connected to the larger diameter portion 55A and extending inward in the width direction. As shown in Figure 7, piecemember 57 that virtually shaped a frustumof a cone is accommodated in the larger diameter portion 55A. The piece member 57 is formed to have a size that can be accommodated in the larger diameter portion 55A while the piece member 57 can move outward in the width direction. Further, the shape of top of the piece member 57 is virtually the same as the internal shape of the concave portion 39, formed in the rear side piece 35. That is, when the piece member 57 is moved outward in the width direction as described below with the first and second formed bodies 14 and 15 connected together, the top of the piece member 57 is fitted into the concave portion 39 so as to inhibit their relative rotations. The smaller diameter portion 55B is set to have virtually the same inner diameter as that of the shaft insertion hole 42 formed in the rear projecting piece 24 of the first formed body 14.

The front intermediate piece 53 comprises a shaft insertion hole 59 penetrating the front intermediate piece 53 in the width direction virtually in its center, and a connecting passage 60 located at a position where the connecting passage 60 does not interfere with the shaft insertion hole 59.

The shaft insertion hole 59 is set to have virtually the same inner diameter as those of the shaft insertion hole 42 and smaller diameter portion 55B. When the rear projecting piece 24 engages with the front projecting piece 50, the shaft insertion hole 59 communicates with the shaft insertion hole 42 and smaller diameter portion 55B. While the shaft insertion hole 59 is in communication with the shaft insertion hole 42 and smaller diameter portion 55B, a pipe-like shaft member 62 is inserted through the shaft insertion holes 42 and 59 and smaller diameter portion 55B (see chain double-dashed lines in Figure 7). This allows the first and second formed bodies 14 and 15 to be connected together so that they are relatively rotatable in the vertical direction. Therefore, the rear projecting piece 24, the front projecting piece 50, and the shaft member 62 constitute connecting means for connecting the bases 20, 20 together so as to be relatively displaceable. The shaft member 62 has an outer diameter that is virtually the same as the inner diameter of the shaft insertion holes 42 and 59 and smaller diameter portion 55B. Further, the shaft member 62 is long enough to allow its opposite ends to project into the large diameter portion 55A when inserted through the holes 42, 59, and 55B (see chain double-dashed lines in Figure 7). When the piece member 57 and the shaft member 62 are assembled together, virtually the entire area of the piece member 57 is accommodated in the larger diameter portion 55A in the state of contacting between the bottom of the piece member 57 and a projecting portion of the shaft member 62. At this time, a gap 58 (see Figure 7) is formed around the shaft member 62.

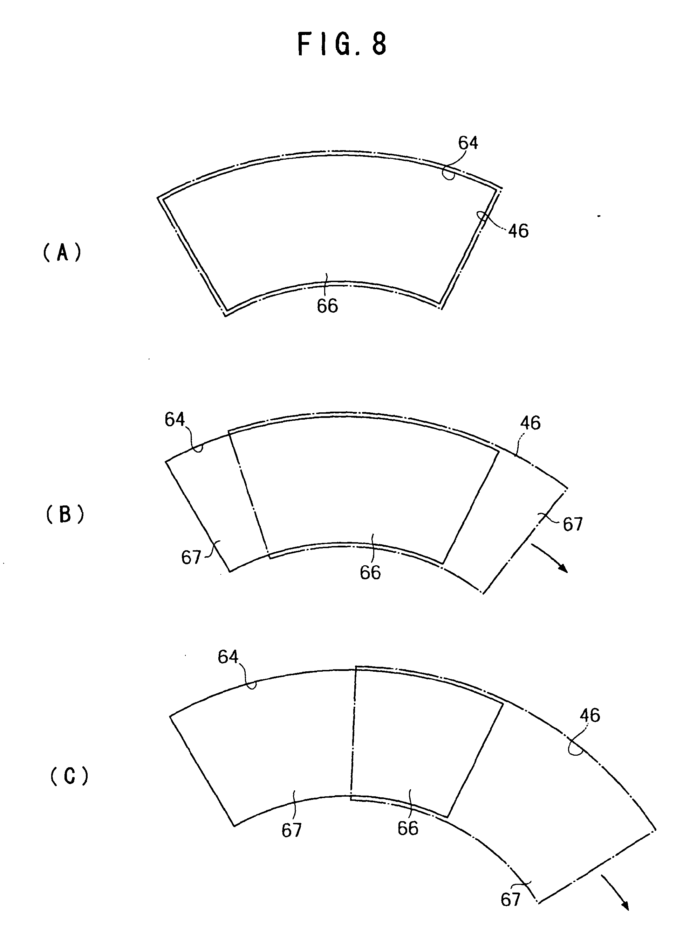

As shown in Figure 7, the connecting passage 60 consists of an upper connecting passage 60A formed in an upper area of the intermediate piece 53, and a lower connecting passage 60B formed in a lower area of the intermediate piece 53 so as not to interfere with the upper connecting passage 60A. The connecting passages 60A and 60B are open to the exterior in opposite side surfaces of the intermediate piece 53. The connecting passage 60A and 60B are shaped like channels extending in the width direction from an open portion 64, bending in the middle of the passages, and then extending rearward. The open portion 64 of the upper connecting passage 60A is open to the top of Figure 7, whereas the open portion 64 of the lower connecting passage 60B is open to the bottom of Figure 7. The open portions 64, 64 have virtually the same shape and size of the open portions 46, 46 in the rear intermediate pieces 37, 37. When the first and second formed bodies 14 and 15 are connected together so as to stand virtually in a line (hereinafter this state will simply be referred to as a "linearly connected position") , the open portions 46 lie opposite the open portions 64 so that each entire area of the open portions are virtually overlapped on each other (see Figure 8(A)). In this state, the upper connecting passages 44A and 60A in the first and second formed bodies 14 and 15 communicate with each other, and the lower connecting passages 44B and 60B communicate with each other. Then, the first and second formed bodies 14 and 15 are relatively rotated to displace from the linearly connected position to a state in which the first and second formed bodies 14 and 15 are connected together while being bent (hereinafter this state will simply be referred to as a "bending connected position"). Then, as shown in Figures 8(B) and 8(C), the area in which the open portions 46, 64 overlap (overlapping area 66) decreases with increasing displacement amount (relative rotation amount). On the other hand, the area in which the open portions 46, 64 do not overlap (non-overlapping area 67) increases gradually. In Figure 8, to avoid confusing in the figure, the contour line of the open portion 64 is shown slightly smaller than that of the open portion 46.

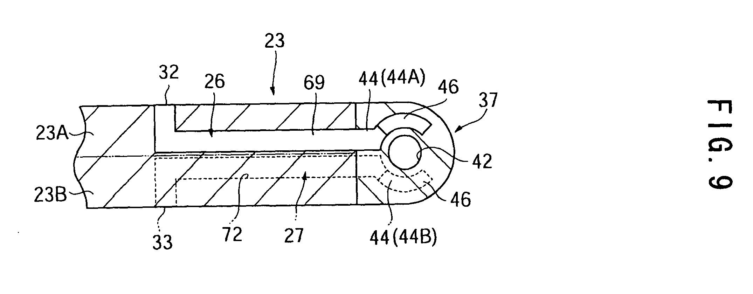

As shown in Figures 7 and 9, the upper channel 26 in the second formed body 15 consists of the upper first supply channel 29, that is in communication with the upper connection passage 60A, an upper second supply channel 69 that is in communication with the upper supply hole 32 which is open on the upper surface of the base 20 of the second formed body 15, and an upper braking channel 70 that is in communication with the member accommodating hole 55 in the lower side of Figure 7. The channels 29, 69, and 70 extend virtually along the longitudinal direction in positions where they do not interfere with one another.

The lower channel 27 and upper channel 26 in the second formed body 15 are virtually symmetrical in the vertical direction and the width direction. The lower channel 27 consists of the lower first supply channel 30 that is in communication with the lower connecting passage 60B, a lower second supply channel 72 that is in communication with the lower supply hole 33 which is open on the lower surface of base 20 of the second formed body 15, and a lower braking channel 73 that is in communication with the member accommodating hole 55 in the upper side of Figure 7.

The braking channels 70 and 73 are in communication with the large diameter portion 55A and particularly with a part of the large diameter portion 55A which is closer to its bottom, that is, to the smaller diameter portion 55B. Specifically, as shown only in the member accommodating hole 55 in the upper side of Figure 7, the braking channels 70 and 73 are allowed to communicate with the gap 58 when the piece member 57 and the shaft member 62 are assembled together.

In the second formed body 15, the rear intermediate piece 37 is placed at three places between the rear side pieces 35 and 35. In each rear intermediate piece 37, the upper connecting passage 44A and the lower connecting passage 44B are formed along positions where they do not interfere with each other, similarly to the connecting channels 60A and 60B in the front intermediate piece 53. The upper connecting passage 44A opens downward in Figure 7 and is in communication with the upper channel 26. That is, the upper connecting channel 44A is in communication with the first supply channel 29, the upper second supply channel 69, and the upper braking channel 70 in order from the top to bottom of the figure. The lower connecting passage 44B opens upward in Figure 7 and is in communication with the upper channel 27. That is, the lower connecting channel 44B is in communication with the lower braking channel 73, the lower second supply channel 72, and the lower first supply channel 30 in order from the top to bottom of the figure.

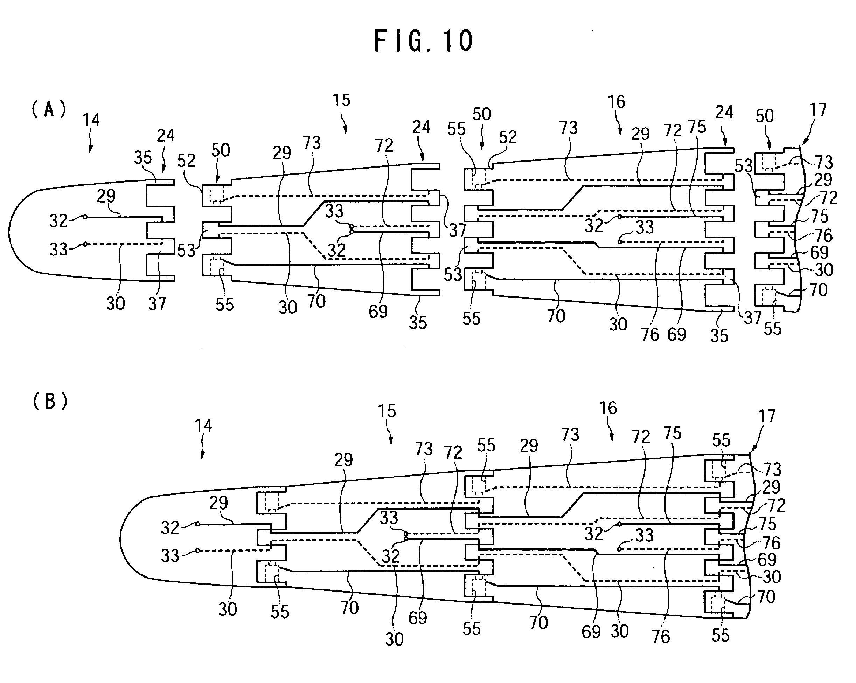

As shown in the conceptual drawing of Figure 10 showing the configuration of the channels, the third formed body 16 has one more front intermediate piece 53 and one more rear intermediate piece 37 than the second formed body 15. In contrast to the second formed body 15, the third formed body 16 has an upper third supply channel 75 that is in communication with the upper supply hole 32 which is open on the upper surface of the base 20, and a lower third supply channel 76 that is in communication with the lower supply hole 33 which is open on the lower surface of the base 20. In Figure 10, channels shown by solid lines are formed in the upper area of the base 20, whereas channels shown by dashed lines are formed in the lower area of the base 20.

A connecting structure for the second and third formed bodies 15 and 16 is the same as that for the first and second formed bodies 14 and 15, already described. Note that, with the second and third formed bodies 15 and 16 connected together (see Figure 10(B)), the upper braking channel 70 and lower braking channel 73 in the second formed body 15 are in communication with the member accommodating holes 55, 55 in the third formed body 16 as previously described.

Although not shown in the figure, the fourth formed body 17 has one more front intermediate piece 53 and one more rear intermediate piece 37 than the forward adjacent formed body 16, and the fifth formed body 18 has one more front intermediate piece 53 and one more rear intermediate piece 37 than the forward adjacent formed body 17. Further, in contrast to the third formed body 16, the fourth formed body 17 has additional supply channels that are in communication with the supply holes 32 and 33, and in contrast to the fourth formed body 17, the fifth formed body 18 has additional supply channels that are in communication with the supply holes 32 and 33. The connected state among the adjacent formed bodies 16, 17, and 18 is virtually the same as that between the second and third formed bodies 15 and 16.

As described above, in the manipulator 10, the supply channels 29, 30, 69, 72, 75, 76, ..., that are in communication with the space 48 (see Figure 2) in each balloon body 21, are formed inside each base 20 so that the channels are independently connected to the rear apparatus main body 12. The channels also allow the formed bodies 14 to 18 to be relatively displaced while maintaining the above state. Further, the upper braking channels 70 in the second to fifth formed bodies 15 to 18 are connected together and to the apparatus main body 12 while allowing the formed bodies 15 to 18 to be relatively displaced. The lower braking channels 73 in the second to fifth formed bodies 15 to 18 are also connected together and to the apparatus main body 12 while allowing the formed bodies 15 to 18 to be relatively displaced.

The apparatus main body 12 includes a heretofore-known pump apparatus that can independently supply normal saline solution to each of the upper channels 26 and lower channels 27. The apparatus main body 12 can control the flow rate and pressure of the normal saline solution ejected.

Operating the apparatus main body 12 enables the manipulator 10 configured as described above to independently supply normal saline solution to the space 48 in any of the balloon bodies 21; the flow rate and pressure of the normal saline solution is under control. Further, when the apparatus main body 12 supplies normal saline solution to the braking channels 70 and 73, the normal saline solution reaches the gap 58 (see Figure 7) in the member accommodating hole 55. The water pressure moves the piece member 57 outward. Then, the piece member 57 is thus fitted into the corresponding concave portion 39 so that the piece member 57 cannot be relatively rotated to concave portion 39. This restricts the relative displacement of the formed bodies 14 to 18 to lock the manipulator 10 in a predetermined position. Therefore, the concave portion 39, the piece member 57, the member accommodating hole 55, and the braking channels 70 and 73 constitute restricting means for restricting the relative displacement of the base 20 using the pressure of the fluid.

Moreover, the adjacent manipulator formed bodies 14 to 18 are displaced from the linearly connected position as a reference position, to the bending connected position. At this time, the open portions 46 and 64 that constitute the joints between the connecting passages 44 and 60 located in the connecting portion, are shifted in the relatively rotating direction, as previously described. Therefore, this forms a non-overlapping area 67 (see Figure 8) where the open portions 46 and 64 do not overlap. Then, the normal saline solution leaks from the non-overlapping area 67 to the exterior of the manipulator 10. The size of the non-overlapping area 67 is designed so that in spite of leakage, the normal saline solution can reach the supply hole 32 and 33 and the member accommodating hole 55. The open portions 46 and 64 form an overlapping area 66 (see Figure 8) even when the adjacent manipulator formed bodies 14 to 18 are displaced in a bending connected position. Consequently, even during the relative displacement, the connecting passages 44 and 60 remain in communication.

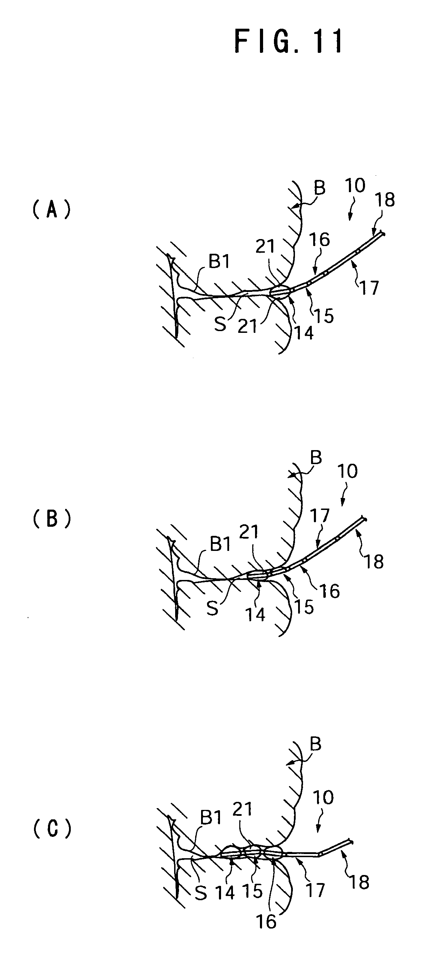

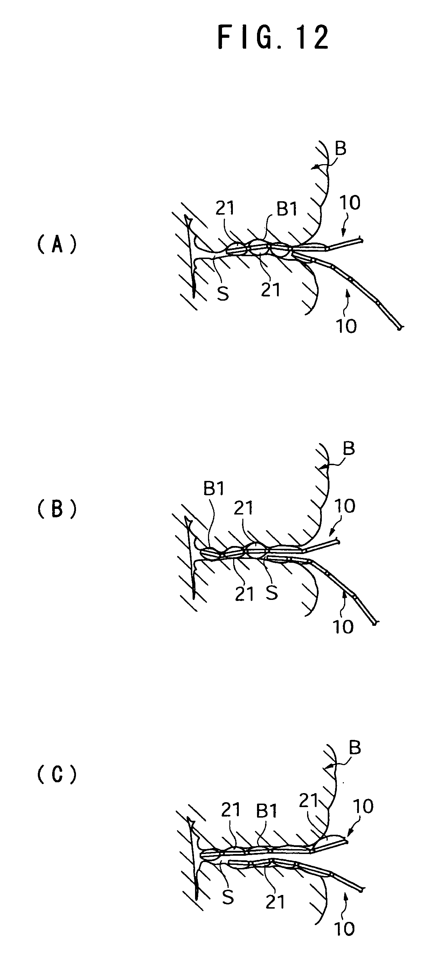

When the manipulator 10 is inserted into for example, the brain, the normal saline solution in the apparatus main body 12 (see Figure 1) starts to be supplied with the front end of the manipulator 10 set near an inlet of a gap S in the brain B as shown in Figure 11. The balloon bodies 21 are then expanded starting from their front ends. In this case, the balloon bodies 21 with a predetermined elasticity softly press a brain tissue B1 around the gap S. The internal pressures of the opposite upper and lower balloon bodies 21, 21 are independently adjusted to regulate a pressing force exerted on the brain tissue B1 by each balloon body 21. When the pressing force on the brain tissue B1 is thus regulated in accordance with the shape of the gap S, the manipulator formed bodies 14 to 18 of the manipulator 10 are displaced between the linearly connected position and the bending connected position under the reaction force from a brain tissue B1 while following the shape of the gap S. The manipulator 10 autonomously advances toward the affected part while pushing through the gap S like a looper. On this occasion, as previously described, part of the normal saline solution leaks from the joints between the manipulator formed bodies 14 to 18 into the gap S. The leaking normal saline solution can be utilized as droplet for the brain tissue B during a surgery. Once the front end of the manipulator 10 reaches the affected part, the apparatus main body 12 supplies the normal saline solution to the braking channels 70 and 73 (see Figure 7 or other figures). Each piece member 57 is moved outward to lock the manipulator 10 to disable the relative displacement of the manipulator formed bodies 14 to 18. Almost simultaneously with this, as shown in Figure 12, another manipulator 10 is inserted into the gap S in a manner similar to that previously described. The balloon bodies 21 are expanded to enlarge the gap S to provide a passage through which the instrument manipulator (not shown) can be inserted. By measuring the internal pressure of each balloon body 21 that is in contact with the brain tissue B1, it is possible to detect, for each relevant site, the pressing force exerted on the brain tissue B1 by the balloon body 21. That is, the manipulator 10 also functions as a pressure sensor that can detect the pressing force on the brain tissue B1.

Accordingly, this embodiment eliminates the need for a robot or the like which advances the manipulator 10 and thus prevents the running out of control of the manipulator 10 associated with the use of a robot. The manipulator 10 can thus be safely moved in the living body. The embodiment also eliminates the need for a wire or motor that serve as a mechanism for displacing the position of the manipulator 10. Consequently, even if the manipulator 10 is broken, electrical leak does not occur in the living body, thus improving safety. The embodiment is also effective in simplifying and miniaturizing the whole system including the manipulator 10. Moreover, the embodiment can be used as means for supplying droplet during an operation or as a pressure sensor that can detect the pressing force on the interior of the living body. The embodiment can thus be suitably used for a narrow gap S such as the interior of the brain B.

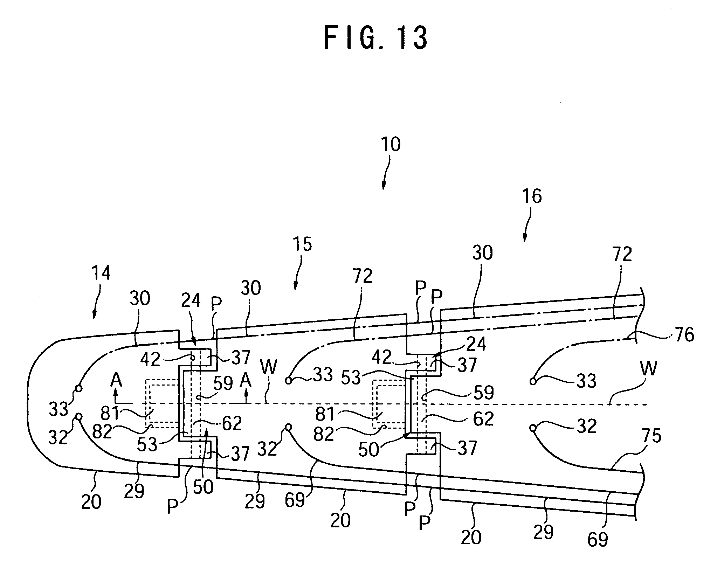

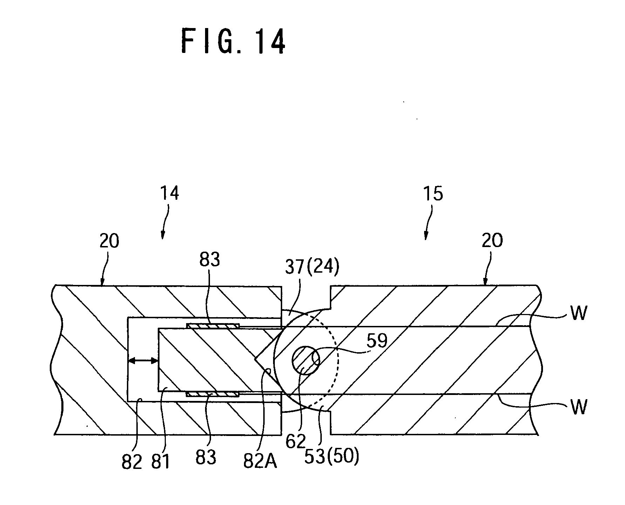

A variation of the embodiment will be described with reference to Figures 13 and 14.

The present variation is characterized by the simplified structure of the manipulator 10. The same reference signs will be used for structural elements in the variation which are substantially the same as or equivalent to those in the above embodiment. The description of these elements will be omitted or simplified. In Figure 13, channels shown by solid lines are formed in the upper area of the base 20. Channels shown by alternate long and short dash lines are formed in the lower area of the base 20.

In the present variation, the rear projecting pieces 24 of the first to fifth formed bodies 14 to 18 have substantially the same shape. The front projecting pieces 50 of the first to fifth formed bodies 15 to 18 have substantially the same shape. No channels for the normal saline solution are provided in the projecting pieces 24 or 50. Flexible pipes P are used to connect together channels with the same reference sign formed in the formed bodies 14 to 18. The channels are connected to the apparatus main body 12 (see Figure 1). Each of the rear projecting pieces 24 is composed of the pair of rear intermediate pieces 37, 37, in which the shaft insertion hole 42 is formed. On the other hand, the front projecting piece 50 is composed of the front intermediate piece 53, positioned between the rear intermediate pieces 37 and 37. The shaft insertion hole 59 is formed in the front intermediate piece 53 so that the shaft insertion hole 59 is in communication with the shaft insertion hole 42 and so that the shaft member 62 can be inserted through the shaft insertion hole 59. In the present variation, the rear projecting piece 24 and the front projecting piece 50 are connected together so as to be relatively rotatable. The pipes P are arranged outside the rear intermediate pieces 37, 37.

An accommodating hole 82 is formed between the rear intermediate pieces 37 and 37 of each of the formed bodies 14 to 18 so that a block-shaped stopper 81 is accommodated in the accommodating hole 82. The stopper 81 is movable through the accommodating hole 82 in the longitudinal direction (lateral direction of Figure 14). A rear end of the accommodating hole 82 is open. A leading edge 82A of the stopper 81 slides out of and back into the open portion. The leading edge 82A is wedged and can slide out of the open portion and engage with a peripheral surface of the intermediate piece 53. A fixed portion 83, 83 (see Figure 14) for a wire W extending toward the apparatus body 12 is provided on both the upper and lower surfaces of the stopper 81. The fixed portions 83, 83 of the formed bodies 14 to 18 are connected together using the wires W. The upper and lower wires W can be wound using a wire winding device (not shown) in the apparatus main body 12. That is, when the wire winding device is activated, the upper and lower wires W are simultaneously pulled toward the apparatus main body 12. The stoppers 81 in the formed bodies 14 to 18 are simultaneously moved rearward. Then, each of the leading edges 82A of the stoppers 81 engages with the front intermediate piece 53 to restrict the relative rotation of the rear projecting piece 24 and front projecting piece 50. Accordingly, the stopper 81, the accommodating hole 82, the fixed portions 83, and the wires W constitute restricting means for restricting the relative rotation of each base 20. On the other hand, when the wire winding device stops its operation to allow the wires W to be relaxed, the restriction on the relative rotation of each base 20 is cancelled. A biasing means such as a spring may be provided between the stopper 81 and the accommodating hole 82 so that when the wire winding device stops its operation, the leading edge 82A of the stopper 81 moves away from the front intermediate piece 53.

In the above embodiment, the present invention is applied to the leading manipulator. However, the present invention is not limited to this but is applicable to other manipulators such as instrument manipulators.

Further, the number of manipulator formed bodies 14 to 18 constituting the manipulator 10 is not limited to the one in accordance with the embodiment. The number of manipulator formed bodies can be increased or reduced. The number of balloon bodies 21 can also be increased or reduced unless the autonomous advancement of the manipulator 10 is hindered.

The fluid supplied to the interior of the balloon body 21 is not limited to the normal saline solution. Another fluid such as a liquid or gas is applicable, as long as that it exerts the above effects and does not affect the livingbody. However, if any balloon body 21 was broken, the use of the normal saline solution alleviates the adverse effect on the living body because a larger amount of normal saline solution is simply supplied to the interior of the living body.

The configuration of each section of the present invention is not limited to the illustrated one. Many variations may be made to the configuration as long as it exerts substantially similar effects.

As described above, the present invention enables the manipulator to advance in the living body without the need for an external propulsive force exerted by a robot or the like. Thus, the manipulator can be autonomously moved in the living body while being prevented from running out of control.

Further, the supply of the fluid activates the manipulator, thus improving the safety of the living body when the manipulator is broken. Moreover, the configuration of the whole system can be simplified to further reduce the size of the system.

### INDUSTRIAL APPLICABILITY

During automatic advancement, the manipulator is prevented from running out of control. The manipulator is therefore suitable as a safe medical instrument.

### BRIEF DESCRIPTION OF THE DRAWINGS

[Figure 1] is a schematic plan view of a manipulator in accordance with the present embodiment;
[Figure 2] is a schematic side view of the manipulator;
[Figure 3] is an exploded perspective view of a first and a second formed bodies;
[Figure 4] is an exploded perspective view as viewed from a direction opposite to that in Figure 3;
[Figure 5] is a schematic transverse sectional view of the first formed body;
[Figure 6] (A) is a vertical sectional view taken along line A-A in Figure 5, and (B) is a vertical sectional view taken along line B-B in Figure 5;
[Figure 7] is a schematic transverse sectional view of the first formed body;
[Figures 8](A) to (C) are conceptual drawings illustrating the relationship between open portions during a displacing operation for bending;
[Figure 9] is a vertical sectional view taken along line C-C in Figure 7;
[Figure 10] (A) is a schematic plan view showing a channel configuration in the state of disassembly of the first, second, and third formed bodies, and (B) is a schematic plan view showing a channel configuration in the state of the connection of the first, second, and third formed bodies;
[Figures 11](A) to (C) are conceptual drawings illustrating a procedure of advancing the manipulator into the brain;
[Figures 12] (A) to (C) are continued drawings of Figure 11 illustrating the procedure of advancing the manipulator into the brain;
[Figure 13] is a schematic plan view showing a channel configuration in the state of the connection of the first, second, and third formed bodies in accordance with a variation of the embodiment; and
[Figure 14] is a sectional view taken along line A-A in Figure 13.

### DESCRIPTION OF SIGNS

10 Manipulator
14 First formed body (manipulator formed body)
15 Second formed body (manipulator formed body)
16 Third formed body (manipulator formed body)
17 Fourth formed body (manipulator formed body)
18 Fifth formed body (manipulator formed body)
20 Base
21 Balloon body
24 Rear projecting piece (connecting means)
26 Upper channel
27 Lower channel
39 Concave portion (restricting means)
44 Connecting passage
48 Space
50 Front projecting piece (connecting means)
55 Member accommodating hole (restricting means)
57 Piece member (restricting means)
62 Shaft member (connecting means)
70 Upper braking channel (restricting means)
73 Lower braking channel (restricting means)
81 Stopper (restricting means)
82 Accommodating hole (restricting means)
83 Fixed portion (restricting means)
W Wire (restricting means)

## Claims

1. A medical manipulator comprising a number of manipulator formed bodies connected together in a longitudinal direction so as to be relatively displaceable, the manipulator advancing in a living body,
wherein at least some of the manipulator formed bodies comprise a base and a pressure section provided on the base to exert a pressing force on tissues in the living body,
the pressure section comprises a space in which a predetermined fluid can be accommodated, the pressing force can be adjusted by regulating an internal pressure of the space, and adjustment of the pressing force allows the manipulator formed bodies to advance autonomously in the living body while being relatively displaced under a reaction force from the tissues.

2. The medical manipulator according to claim 1, wherein the pressure section comprises a balloon body that expands and deflates in response to an increase and decrease in the amount of fluid accommodated.

3. The medical manipulator according to claim 1 or 2, wherein the pressure section is provided in each of the manipulator formed bodies and the pressing force of each pressure section is independently adjustable.

4. The medical manipulator according to claim 1, 2, or 3, wherein the pressure section is provided on both a upper and lower surfaces of the base so as to press the tissues upward and downward with respect to the base.

5. The medical manipulator according to any of claims 1 to 4, wherein a channel is formed in the base so that the channel is in communication with a space in the pressure section.

6. The medical manipulator according to claim 5, wherein connecting means is provided between the bases to connect the bases so that the bases can be relatively displaced, and a connecting passage is formed in the connecting means to allow said channels formed in the bases to be in communication with each other.

7. The medical manipulator according to claim 6, wherein the connection passage is configured to keep the channels in communication with each other even when the bases are relatively displaced.

8. The medical manipulator according to claim 6 or 7, wherein the fluid is normal saline solution, and the connecting passage allows leakage of part of the normal saline solution passing through the connecting passage so as to supply the normal saline solution to the interior of the living body.

9. The medical manipulator according to any of claims 1 to 8, wherein restricting means are provided between the manipulator formed bodies so as to restrict the relative displacement of the manipulator formed bodies.
